# EUROPÄISCHE PATENTSCHRIFT

(11) **EP 1 315 740 B1**
(45) Veröffentlichungstag und Bekanntmachung des Hinweises auf die Patenterteilung: **23.09.2009**
(21) Anmeldenummer: 01967286.4
(22) Anmeldetag: 18.08.2001
(51) Int. Cl.: C07K 1/113, C07K 1/36

(54) **VERFAHREN ZUR AUFREINIGUNG VON REKOMBINANTEN ALS UNLÖSLICHE AGGREGATE EXPRIMIERTE VARIANTEN DES BET V 1 ALLERGENS**
METHOD FOR PURIFYING DIFFERENT FORMS OF RECOMBINANT BET V 1 ALLERGEN EXPRESSED AS INSOLUBLE AGGREGATES
PROCEDE DE PURIFICATION DE DIFFERENTES FORMES DE L'ALLERGENE BET V 1 RECOMBINANTES EXPRIMEES EN TANT QU'AGREGATS INSOLUBLES

(30) Priorität: 08.09.2000 DE 10044360
(43) Veröffentlichungstag der Anmeldung: 04.06.2003
(73) Patentinhaber: Merck Patent GmbH, 64293 Darmstadt (DE)
(72) Erfinder: SUCK, Roland, 22301 Hamburg (DE); CROMWELL, Oliver, 21465 Wentorf (DE); FIEBIG, Helmut, 21493 Schwarzenbek (DE)
(86) Internationale Anmeldenummer: PCT/EP2001/009552
(87) Internationale Veröffentlichungsnummer: WO 2002/020559

(56) Entgegenhaltungen:
- EP-A- 0 432 419
- WO-A-98/14467
- LORENZ A R ET AL: "Recombinant food allergens" JOURNAL OF CHROMATOGRAPHY. BIOMEDICAL APPLICATIONS, ELSEVIER, AMSTERDAM, NL, Bd. 756, Nr. 1-2, 25. Mai 2001 (2001-05-25), Seiten 255-279, XP004249671 ISSN: 0378-4347

## Beschreibung

Die Erfindung betrifft ein Verfahren zur Solubilisierung und Aufreinigung von rekombinanten Proteinen, die in bakteriellen Wirtszellen exprimiert und als unlösliche Aggregate (*inclusion bodies*) abgelagert werden. Die Aufreinigung basiert auf der Überführung besagter "inclsuion bodies" in lösliche Formen unter Verwendung von organischen Denaturierungsreagenzien und Einsatz chromatographischer Verfahren. Hierbei werden anorganische, alkalische, salzhaltige Laufmittel ausgewählt, die es nach erfolgter Aufreinigung ermöglichen, die rekombinanten Proteine nach Neutralisation in für den medizinschen Gebrauch direkt einsetzbarer und physiologisch akzeptabler Form zur Verfügung zu stellen. Das Verfahren ist insbesondere geeignet, für die Aufreinigunng von Allergenen und Allergenfragmenten.

Das erfindungsgemäße Verfahren wird unter Bedingungen durchgeführt, die für Pharmazeutika notwendig sind (GMP). Die pharmazeutischen Wirkstoffe können direkt nach Solubilisierung als Parenteralia verwendet werden. Die nach dem erfindungsgemäßen Verfahren hergestellten bevorzugten rekombinanten Allergene oder Allergenvarianten können sowohl zur verbesserten Therapie als auch zur Diagnose von allergischen Erkrankungen Verwendung finden.

Es ist ein generelles Problem bei der Herstellung rekombinanter Proteine durch bakterielle bzw. prokaryontische Wirtszellen, wie beispielsweise E. coli, daß die exprimierten Proteine nicht die richtige, respektive native Faltung aufweisen, die sie in der Regel benötigen, um volle biologische Aktivität zu zeigen. Die falsche Faltung führt häufig dazu, daß die eine Reihe von Proteinen nur in im Expressionsmedium unlöslicher Form vorliegen, bzw. in Form von Aggregaten abgelagert werden, welche als sogenannte "*inclusion bodies*" Einzug in die wissenschaftliche Literatur gehalten haben. Die Bildung besagter "inclusion bodies" wirkt sich auf die Reinigung und notwendige lösliche Verfügbarkeit des exprimierten rekombinanten Proteins ungünstig aus (Marston et al., 1986, Biochem J. 240: 1-12). Um die in Bakterien abgelagerten rekombinanten Proteine reinigen zu können, werden chromatographischen Laufmitteln häufig denaturierende Substanzen, wie z.B. Harnstoff oder Guanidiniumhydrochlorid, zugesetzt. Diese Zusätze sind jedoch nicht mit jedem Trennprinzip vereinbar, wie z.B. der hydrophoben Interaktionschromatographie. Zudem können durch chemische Reaktionen die Produkte nachteilig verändert werden, wie z.B. durch Cyanat, das sich in Harnstofflösungen bilden kann. Der Entzug des Denaturierungsmittels und damit die mögliche Renaturierung erfolgt üblicherweise durch Dialyse, Diafiltration oder Gelfiltration. Diese Prozesse sind nicht nur langwierig, sondern führen häufig zu einer erneuten Präzipitation der Produkte. Der analytische Nachweis einer vollständigen Entfernung von o. g. Denaturierungsmitteln im Endprodukt ist schwierig. Die oben dargestellte Problematik ist besonders bei der Herstellung und Aufreinigung von rekombinanten Allergenen und Allergenvarianten von Bedeutung.

Allergien vom Typ 1 haben in den letzten Jahrzehnten weltweit dramatisch zugenommen. Bis zu 20% der Bevölkerung in industrialisierten Ländern leiden unter Beschwerden, wie allergischer Rhinitis, Konjunktivitis oder Bronchialasthma, die durch in der Luft befindliche Allergene (Aeroallergene), die von unterschiedlichen Quellen wie Pflanzenpollen, Milben, Säugetieren (Katzen, Hunden, Pferden) und Schimmelpilzen freigesetzt werden, hervorgerufen werden. Schwerwiegende Allergien können auch durch Stiche von Insekten, wie z.B. von Bienen und Wespen, ausgelöst werden.

Bei den Typ 1 - Allergie auslösenden Substanzen handelt es sich um Proteine, Glykoproteine oder Polypeptide. Diese Allergene reagieren nach Aufnahme über die Schleimhäute oder nach Stichen den bei sensibilisierten Personen an der Oberfläche von Mastzellen gebundenen IgE-Antikörpern. Werden zwei oder mehr IgE-Antikörpem durch ein Allergen miteinander vernetzt, führt dies zur Ausschüttung von Mediatoren (z.B. Histamin, Prostaglandinen) und Zytokinen durch die Effektorzelle und damit zur Auslösung der allergischen Sypmtome.

Mit Hilfe von cDNA-Sequenzen ist es möglich, rekombinante Allergene herzustellen, die in der Diagnostik und Therapie von Allergien Verwendung finden können (Scheiner und Kraft, 1995, Allergy 50, 384-391). Besondere Bedeutung können rekombinante Allergene bei diagnostischen Methoden, die im Vergleich zu den konventionellen Extrakten die Identifizierung individueller IgE-Sensibilisierungsspektren ermöglichen, erlangen. Darüber hinaus sind gezielte gentechnische Veränderungen der rekombinanten Allergene möglich, wodurch ein reduziertes allergenes Potenzial bei unveränderter Reaktivität mit den regulatorischen T-Helferzellen erreicht werden kann (Schramm et al., 1999, J. Immunol. 162 (4): 2406-2414; Valenta et al., 1999, Biol. Chem. 380: 815-24; Singh et al., 1999, Int. Arch. Allergy Immunol. 119: 75-85). Solche Allergenvarianten sind aussichtsreiche zukünftige Kandidaten für die spezifische Immuntherapie der Typ 1-Allergie. Insbesondere, letztere genannte veränderte Allergene sind Gegenstand des erfindungsgemäßen Verfahrens.

In diesem Zusammenhang ist die Produktion von rekombinanten Allergenen und Allergenvarianten in den häufig verwendeten bakteriellen Expressionssystemen von besonderer Bedeutung. Diese Systeme bieten im Vergleich zu eukaryontischen Systemen den Vorteil, dass bereits nach kurzer Expressionszeit hohe Produktausbeuten zu erhalten sind. Zudem sind sie aus pharmakologischer Sicht weitaus risikoloser in Hinsicht auf virale Kontaminationen und Onkogene. Jedoch werden bei ihrer rekombinanten Herstellung in bakteriellen Expressionssystemen die Hauptallergene verschiedener Quellen, wie z.B. die Graspollenallergene Gruppe 1 (Vrtala et al., 1996, J. Allergy Clin. Immunol. 97: 781-7) und 13 (Suck et al., 2000, Clin. Exp. Allergy 30: 324-332), das Milbenallergen Der f 2 (Iwamoto et al., 1996, Int. Arch. Allergy Immunol. 109: 356-61) sowie die Insektengiftallergene Phospholipase A2 und Hyaluronidase (Soldatova et al., 1998, J. Allergy Clin. Immunol. 101: 691-8; Kuchler et al., 1989, Eur. J. Biochem. 184: 249-254) als *inclusion bodies* in der Wirtszelle abgelagert.

Auch die Allergenvarianten, wie z.B. Fragmente und Multimere des Birkenpollenhauptallergens Bet v 1 (Fragment A, Fragment B, Bet v 1- Trimer) verhalten sich in dieser Weise, obwohl das unveränderte rekombinante Bet v 1 - Allergen weitgehend löslich ist (Hoffmann-Sommergruber et al., 1997, Prot. Expr. Purific. 9: 233-39; van Hage-Hamsten et al., 1999, J. Allergy Clin. Immunol. 104: 969-7).

Es bestand somit die Aufgabe, ein Verfahren zur Verfügung zu stellen, dass es ermöglicht, rekombinante, als "inclusion bodies" vorliegende Proteine, insbesondere Allergene, bzw. Proteine mit allergener Wirkung, in einfacher und wirkungsvoller Weise aufzureinigen unter Vermeidung der oben genannten Nachteile der Verfahren des Standes der Technik. Überdies bestand die Aufgabe, die Proteine in der Weise zu gewinnen, dass sie während und insbesondere am Ende der Aufreinigung in einer Form vorliegen, die es ermöglicht, dass sie unmittelbar und ohne weitere Prozessierung für die medizinische oder diagnostische Verwendung zur Verfügung gestellt werden können.

Bei der vorliegenden Erfindung handelt es sich um ein biochemisches Reinigungsverfahren, das über ein effizientes ein - oder mehrstufiges, vorzugsweise ein - bis dreistufiges Reinigungsverfahren von rekombinanten varianten des Birkenpollenhauptallergens Bet v1, welche als inclusion bodies nach ihrer Exprimierung vorliegen, unter Verwendung von im wesentlichen ungepufferten alkalischen Laufmitteln zur reinen Darstellung der Proteine führt.

Bei der Erfindung handelt es sich auch um ein Verfahren, bei dem die rekombinanten Proteine abschließend nach dem letzten Reinigungsschritt durch rasche Neutralisierung in Lösung bleiben. Gleichzeitig ergibt sich dadurch die Schaffung eines physiologischen Milieus.

Als Allergenvarianten im Sinne der Erfindung werden Fragmente, Multimere, wie z.B. Dimere oder Trimere, aber auch Modifikationen der ürsprünglichen Allergene oder ihrer Fragmente / Multimere verstanden. Letztere weisen im Sinne der Erfindung Insertionen, Deletionen oder Substitutionen von einzelnen oder mehreren Aminosäuren auf.

Vorzugsweise werden rekombinante, in der Natur nicht vorkommende Allergenvarianten eingesetzt, die keine oder eine herabgesetzte IgE - Aktivität, jedoch eine konservierte T - Zell Aktivität besitzen. Das Verfahren eignet sich für die Gewinnung der Allergenvarianten des Birkenpollenhauptallergens Bet v1, nämlich der rekombinanten Fragmente A (Aminosäure 1-74) und B (Aminosäure 75-164) sowie eines rekombinanten Trimers. Nach diesem Verfahren hergestellte rekombinante Allergene und Allergenvarianten sind vorzüglich geeignet, um für die spezifische lmmuntherapie und Diagnostik von allergischen Erkrankungen eingesetzt zu werden.

Gegenstand der Erfindung ist somit Verfahren zur Herstellung von gereinigten, rekombinanten Varianten des Birkenpollenhauptallergens Bet v1 in löslicher, biologisch aktiver und in physiologischem, unmittelbar für den medizinischen Einsatz geeigneten Medium vorliegender Form aus nach bakterieller Expression erhaltenen, im Expressionsmedium unlöslichen Proteinaggregaten ("inclusion bodies"), welches durch folgende Schritte ausführt zu beschreiben ist:
(i) Aufnahme der abgetrennten unlöslichen Proteinaggregate in organischen Denaturierungs - Reagenzien,
(ii) Aufreinigung der Lösung aus (i) mittels mindestens eines chromatographischen Schrittes unter Verwendung von im wesentlichen anorganischen ungepufferten alkalischen, salzhaltigen Laufmitteln und
(iii) Neutralisation der nach dem letzten Aufreinigungsschritt erhaltenen, das gelöste, renaturierte und biologisch aktive Protein enthaltenden alkalischen Lösung.

Für die Aufreinigung werden die primär gebildeten unlöslichen Aggregate zunächst in an sich bekannten Denaturierungsreagenzien in Lösung gebracht und dadurch denaturiert. Vorzugsweise wird Harnstoff, insbesondere 5 bis 10 M Harnstoff, vorzugsweise 8 M Harnstoff eingesetzt. Alternativ können auch andere Mittel, wie beispielsweise höher konzentrierte Natron- oder Kalilauge (0.2 M bis 1 M) oder das oben erwähnte Guanidiniumhydrochlorid Verwendung finden.

In einem weiteren Schritt wird das gelöste, denaturierte Protein an ein geeignetes chromatographisches Material gebunden. Hierbei sind in erster Linie Ionenaustauscher, vorzugsweise Anionenaustauscher, geeignet.

Als Laufmittel der Chromatographie wird erfindungsgemäß eine im wesentlichen anorganische ungepufferte, alkalische, salzhaltige Lösung eingesetzt. Geeignete Laufmittel sind Lösungen von beispielsweise NaOH oder KOH zusammen mit NaCl oder KCl. Gegebenenfalls und in einer bevorzugten Ausführungsform ist dem Laufmittel Natrium- oder Kaliumhydrogencarbonat zur Möglichkeit der pH-Wert Absenkung beigefügt. Bevorzugte Laufmittel enthalten NaOH, NaCl, bzw. NaOH, NaCl und NaHCO₃. Die wäßrige alkalische Lösung weist dabei erfindungsgemäß einen Gehalt von NaOH bzw. KOH von 5mM bis 50 mM (mol / L), vorzugsweise von 15 bis 25 mM, insbesondere 20 mM auf. Die Salzkonzentration (z.B. NaCl) liegt anfangs relativ niedrig, so dass das Protein am Ionenaustauschermaterial fest bindet. Erfindungsgemäße Anfangs-Salzkonzentrationen liegen zwischen 5 und 50 mM, vorzugsweise zwischen 15 und 30 mM, insbesondere bei 20 mM. Hierdurch wird das Denaturierungsmittel sowie Verunreinigungen, die nicht oder nicht fest genug am Chromatographie-material binden, entfernt. Falls NaHCO3 zugesetzt wird, weist dieses einen Gehalt von 5 bis 15 mM, vorzugsweise von 11 mM auf.

Gegenstand der Erfindung ist somit also ein entsprechendes Verfahren, bei welchem das Denaturierungs - Reagenz aus der Denaturierungslösung entfernt wird, in dem man das gelöste rekombinante Protein an ein Chromatographie-Material bindet und die Denaturierungs - Lösung gegen ein im wesentlichen anorganisches ungepuffertes, alkalisches Laufmittel austauscht, welches eine Salzkonzentration aufweist, die eine Bindung des Proteins an das besagte Austauschermaterial gewährleistet.

Um das gebundene rekombinante Protein zu eluieren wird gemäß des erfindungsgemäßen Verfahrens bei gleichbleibenden anderen Bedingungen mittels eines Gradienten sukzessive eine höhere Salzkonzentration (NaCl bzw. KCl) bereitgestellt. Vorzugsweise wird ein linearer Gradient zwischen etwa 20 mM NaCl. (KCl) (Startwert) und 0,5 M NaCl (Endwert) verwendet. Hierdurch wird nicht nur das gewünschte Protein wieder gewonnen, sondern es wird von weiteren Verunreinigungen (z.B. anderen Proteinen) getrennt.

Somit ist Gegenstand der Erfindung ein entsprechendes Verfahren, welches dadurch gekennzeichnet ist, daß das gebundene rekombinante Protein durch Erhöhung der Salzkonzentration eluiert und dabei von Verunreinigungen befreit wird, wobei vorzugsweise die Erhöhung der Salzkonzentration mittels eines Gradienten bewerkstelligt wird.

In einigen Fällen kann es ausreichen, keinen weiteren Reinigungsschritt anzuschließen, da das eluierte Protein entsprechend den Anforderungen ausreichend gereinigt ist. In solchen Fällen schließt sich direkt der unten näher beschriebene Neutralisationsschritt an, um ein gebrauchsfertiges und aktives Protein zu erhalten.

Andernfalls erfolgt gemäß dem Verfahren ein weiterer Chromatographieschritt. Hierbei sind generell alle bekannten Chromatographien einsetzbar. Im einzelnen wird sich dies nach den chemisch-physikalischen Eigenschaften des jeweiligen zu reinigenden Proteins richten. Im Falle von Allergenen und Allergenvarinaten des Typs Bet v1, erfolgt die weitere Aufreinigung mittels hydrophober Interaktionschromatographie. Alternativ kann stattdessen auch eine Gelfiltration erfolgen.

Eine bevorzugte Ausführungsform des erfindungsgemäßen Verfahrens ist ein Dreistufenverfahren, welches Ionenaustauscher-, hydrophober Interaktionschromatographie und Gelfiltration, vorzugsweise in der genannten Reihenfolge, umfaßt. Diese bedeutet aber keine Einschränkung des erfindungsgemäßen Verfahrens.

Gegenstand der Erfindung ist also somit ferner ein entsprechendes Verfahren, welches mindestens einen weiteren Chromatographieschritt, vorzugsweise hydrophobe Interaktionschromatographie und / oder Gelfiltration, aufweist.

Erfindungswesentlich ist all diesen Aufreinigungsschritten, dass sie mit der gleichen qualitativen und quantitativen Zusammensetzung der Elutions- oder Laufmittel arbeiten, welche oben bereits ausführlich beschrieben wurden: NaOH, NaCl und vorzugsweise NaHCO₃, bzw. die entsprechenden Kaliumderivate. Lediglich die Konzentration der Kochsalzkomponente kann bei den verschieden Schritten variabel gestaltet sein. So wird beispielsweise bei der hydrophoben Interaktionschromatographie ein NaCl (KCl) - Gradient von hoher (1- 3 M, vorzugsweise 2 M) zu niedriger (30-10 mM, vorzugsweise 20 mM) Konzentration eingesetzt. Die Endkonzentration richtete sich dabei letztlich nach der gewünschten Salzkonzentration nach dem letzten Aufreinigungsschritt. Diese Konzentration soll erfindungsgemäß die Konzentration einer physiologisch verträglichen Lösung sein. Ist beispielsweise die Gelfiltration bei dem bevorzugten Drustufenreinigungs-Verfahren der letzte Reinigungsschritt, wird etwa 150 mM NaCl verwendet. Letztlich kann die gewünschte Endkonzentration an Salz nach dem letzten Schritt auch durch Verdünnen oder Hinzugabe von Salz gesteuert werden, wobei dies Wiederum von den chromatographierten Mengen an Zielprotein abhängt.

Nach dem letzten Aufreinigungsschritt - in der bevorzugten Ausführungsform des Verfahrens die Gelfiltration - erfolgt die Neutralisation der Lösung bzw. Renaturierung des Proteins durch Hinzugabe von verdünnter Säure, vorzugsweise HCl, unter Einstellung des pH-Wertes von 6,5 bis 8,0. Die Lösung mit dem aktiven rekombinanten Protein kann nunmehr, ggf. nach nachträglicher Einstellung des Salzgehaltes (z.B. Na+) und Proteingehaltes in formulierter Form direkt eingesetzt werden.

Gegenstand der Erfindung ist somit ein entsprechendes Verfahren, welches dadurch gekennzeichnet ist, daß die Neutralisation nach erfolgter Aufreinigung in einem pH-Wert Bereich zwischen 6.5 bis 8.0 durchgeführt wird, wobei vorzugsweise verdünnte Säure, insbesondere verdünnte HCl, eingesetzt wird.

Im folgenden wird das erfindungsgemäße Verfahren am Beispiel der Gewinnung der Allergenvarianten des Birkenpollenhauptallergens Bet v 1, nämlich der rekombinanten Fragmente A (Aminosäure 1-74) und B (Aminosäure 75-164) sowie eines rekombinanten Trimers, näher beschrieben:
Für die Aufreinigung werden die primären unlöslichen Aggregate denaturiert, vorzugsweise in 8 M Harnstoff. Alternativ können auch andere Mittel eingesetzt werden, beispielsweise 0.2 M Natronlauge.

Der erste chromatographische Aufreinigungschritt erfolgt mittels Anionaustauschschromatographie, beispielsweise an Source Q. Hierbei werden die meisten Allergene oder Allergenvarianten an den Träger gebunden und von der initialen Denaturierungslösung in das Laufmittel überführt. Das alkalische Laufmittel bewirkt, das die Proteine in Lösung verbleiben. Durch NaCl-Gradientenelution erfolgt eine partielle Abtrennung von bakteriellen Verunreinigungen und Wirkstofffragmenten.

In zwei weiteren Aufreinigungsschritten, einer hydrophobe Interaktionschromatographie und einer Gelfiltration, werden die vorgereinigten und äquilibrierten Allergene oder Allergenvarianten im wesentlichen von noch verbliebenen bakteriellen Verunreinigungen abgetrennt. Hierzu werden grundsätzlich die gleichen Laufmittelsubstanzen benutzt, bestehend aus niedermolarer Base und einem variierenden Anteil anorganischen Salzes. Gegenstand der Erfindung ist somit ein spezielles Laufmittelsystem, worin die zur reinigenden rekombinanten Proteine während der Reinigung schonend in Lösung gehalten werden und das zu einer effektiven Reinigung führt.

Nach dem letzten Chromatographieschritt können die gereinigten rekombinanten Proteine erfindungsgemäss durch einfache Neutralisation der im Laufmittel vorhanden Base mit einer entsprechenden Säure in löslicher Form gewonnen oder renaturiert werden. Bei geeigneter Wahl der Konzentrationen der Laufmittelzusätze entsteht eine für Parenteralia geeignete physiologische Lösung. Die Identifikation der gereinigten rekombinanten Allergene oder Allergenvarianten erfolgt über ihre bekannten physikalischen, chemischen, immunologischen oder biologischen Eigenschaften, insbesondere mittels isoelektrischer Fokussierung, SDS-PAGE und spezifischer monoklonaler Antikörper sowie IgE-Antikörpern von Allergikern. Die Prüfung des Lösungsmittels erfolgt durch pH-Wert Messung und Quantifizierung der Na⁺- und Cl⁻ - sowie ggfs. CO³⁻ -Konzentration. Diese Verfahren sind allgemein bekannt und beschrieben. Die Ausbeute der erfindungsgemäss gereinigten und solubilisierten rekombinanten Allergene oder Allergenvarianten beträgt 75-95 % bezogen auf das primäre Ausgangsprotein.

Die so hergestellten Allergen-Komponenten können bei der In vivo- und In vitro-Diagnostik im Rahmen einer Allergen-Komponenten auflösenden Identifizierung des Patienten-spezifischen Sensibilisierungsspektrums eingesetzt werden ebenso wie zur spezifischen Immuntherapie von Allergien. Weiterhin können durch Umsetzung der gereinigten rekombinanten Proteine pharmazeutische Zubereitungen in Form von Depotpräparaten bereitet werden.

Eine bevorzugte Ausführungsform des erfindungsgemäßen Verfahrens ist in der folgenden Darstellung (Tab. 1) schematisiert:

**Tab. 1**

| Isolierung von *inclusion bodies* |
|---|
| |
| Denaturierung z.B. in 8M Harnstoff |
| |
| Anionaustauschchromatographie, z.B. Source Q |
| *Lösung A: 20 mM NaOH, 20 mM NaCl, 11 mM NaHCO₃* |
| *Lösung B: 20 mM NaOH, 0.5 M NaCl, 11 mM NaHCO₃* |
| |
| (optional) Hydrophobe Interaktionschromatographie, z.B. Source PHE |
| Lösung A: 20 mM NaOH, 2 M NaCl, 11 mM NaHCO₃ |
| Lösung B: 20 mM NaOH |
| |
| (optional) Gelfiltration, z.B. Superdex 75 |
| Lösung A: 10 mM NaOH, 11 mM NaHCO₃ |
| 148.4 mM NaCl |
| |
| Renaturierung / Formulierung |
| *Zielprotein in 10 mM NaOH, 148.4 mM NaCl, 11 mM NaHCO₃* |
| *+ Zugabe von 100 mM HCl ad 10 mM* |
| = *solubles Zielprotein in 0.154 M Na+-Lösung* |

Zusammenfassend kann also festgestellt werden: Die vorliegende Erfindung ermöglicht also durch das zur Verfügung gestellte erfindungsgemässe Verfahren, welches sich durch die spezielle Zusammensetzung der Chromatographielaufmittel, die Wahl der Chromatographiemedien sowie die spezielle Einstellung des pH-Wertes und damit des Salzgehaltes, eine technologisch und pharmakologisch umsetzbare Produktionsmethode zur Gewinnung von hochreinen, löslichen rekombinanten Allergenen und Allergenvarianten, die primär als unlösliches Aggregat hergestellt werden, mit geringem Arbeits- und Zeitaufwand. Da die Bedingungen, unter denen die rekombinanten Proteine gereinigt und renaturiert bzw. solubilisiert werden, im Vergleich zu bekannten Methoden schonend und pharmakologisch geeignet sind, können die Wirkstoffe sowohl für eine Diagnostik als auch für eine parenteral Therapie von allergischen Erkrankungen eingesetzt werden.

### Beispiel:

### Gewinnung von löslichen Allergenvarianten des Bet v 1 (therapeutisch wirksamee Allergenvarianten des rekombinanten Bet v 1, Fragment A und B sowie dem Bet v 1- Trimer)

Die nach Standardverfahren gereinigten *inclusion bodies* der Allergenvarianten werden vorzugsweise in 8 M Harnstoff, 20 mM Tris/HCl denaturiert. Nach der vollständigen Denaturierung wird eine Filtration, vorzugsweise 0.22 - 0.45 µm, oder eine Zentrifugation, vorzugsweise 5-15 min bei 10000-20000 xg, durchgeführt. Die geklärte Lösung wird zur Ionenaustauschchromatographie mit Source 15Q (Pharmacia) eingesetzt, wobei das Trägermaterial mit alkalischer Lösung, vorzugsweise 20 mM NaOH, 11 mM NaHCO₃ und 20 mM NaCl, äquilibriert ist. Das Trägermaterial muss bei pH-Werten von bis 12.0 stabil sein. Der relativ hohe pH-Wert der Startlösung bewirkt, das nahezu alle Zielproteine an den Anionenaustauscher binden. Ausnahmen bilden äusserst seltene Proteine, deren Isoelektrischer Punkt oberhalb von 11.0 liegt. Durch das Spülen der immobilisierten Proteine mit Startlösung werden die Denaturierungslösung (z.B. 8M Harnstoff) sowie pharmakologisch ungünstige Puffersubstanzen (z.B. Tris) effektiv entfernt. Durch dieses Verfahren wird gewährleistet, daß bei Erhaltung der Löslichkeit der rekombinanten Proteine ein Wechsel zu einem Lösungsmittel, welches die nachfolgenden Separationsschritte ermöglicht, stattfindet. Die anschliessende Elution erfolgt mit ansteigendem NaCl-Gradienten, z.B. von 20mM NaOH, 11 mM NaHCO₃ 20 mM NaCl zu 20mM NaOH, 11 mM NaHCO₃,0.5 M NaCl, und bewirkt eine Abtrennung von Verunreinigungen (Wirtszellproteinen) und Wirkstofffragmenten.

Der nächste, Chromatographieschritt stellt eine hydrophobe Interaktionschromatographie dar (nur für die Fragmente). Dazu wird das Eluat aus Schritt 1 mit einer hochmolaren Salzlösung, z.B. 5 M NaCl und 20 mM NaOH, 11 mM NaHCO₃ derart eingestellt, dass das Zielprotein bindet. Die Elution des gebundenen Zielproteins erfolgt mit salzarmer oder salzfreier alkalischer Lösung, z.B. 20 mM NaOH. Der zur hydrophoben Interaktionschromatographie eingesetzte Träger muss ebenfalls alkalistabil bis pH 12 sein, wie z.B. Source PHE.

Als dritter Schritt wird eine Gelfiltration, z.B. Superdex 75, unter alkalischen Bedingungen durchgeführt. Die Chromatographielösung wird derart gewählt, das eine Neutralisation der im Laufmittel zugesetzten Base zu der gewünschten Endformulierung führt, wie z.B. 10 mM NaOH, 11 mM NaHCO₃ und 148.4 mM NaCl.

Das Eluat der Gelfiltration wird zum Abschluss mit einer zur verwendeten Base korrespondierenden Säure neutralisiert, wodurch einerseits ein neutraler pH-Wert eingestellt und damit das Protein in eine lösliche Form überführt oder renaturiert wird und anderseits durch Neutralisation der gewünschte Salzgehalt eingestellt wird. So wird z.B. eine Gelfiltrationslösung aus 10 mM NaOH, 11 mM NaHCO₃ und 148.4 mM NaCl durch Zugabe von 1/10 (v/v) 100 mM HCl in physiologische Kochsalzlösung überführt. Die Prüfung des Lösungsmittels erfolgt durch einfache Messung des pH-Wertes sowie Na⁺- Quantifizierung. Das Verfahren beinhaltet somit erfindungsgemäss minimale Probenbehandlungen, kurze Probenstandzeiten, die Verwendung von ausschließlich pharmakologisch kompatiblen Substanzen, die Kompatibilität eines einzigen Laufmittels mit diversen Trennprinzipien, und die Umgehung von langwierigen und u.U. nicht validierbaren Verfahren wie Dialysen. Zudem verhindert die Natronlauge, die als effektives Bakteriostatikum bekannt ist, dass die in ihr vorhandenen Proteine durch Mirkroorganismen degradiert bzw. verunreinigt werden. Ebenso werden Endotoxine, die bei bakteriellen Expressionen problematisch sein können, effektiv entfernt bzw. degradiert. Die Reihenfolge und die Anzahl der oben beschriebenen Chromatographieschritte kann je nach den spezifischen physiochemischen Eigenschaften der Zielproteine verändert werden. Eine Übersicht über die Lösungen ist Tab. 2 zu entnehmen.

### Neutralisierung/Solubilisierung nach Gelfiltration:

- langsame Zugabe von 1/10 Volumen (bezogen auf die Vorlage) von 100 mM HCl.
z.B. in 100 ml Ausgangslösung enthalten:
10 mM NaOH, 148.4 mM NaCl, 11 mM NaHCO3
nach Neutralisation 110 ml:
11 mM NaHC03 *10/11=10 mM NaHCO3
10 mM NaOH/HCl (NaCl) * 10 /11 = 9.1 mM NaCl
148.4 mM NaCl *10/11 = 134.9 mM NaCl

Die fertige Lösung setzt sich demnach zusammen aus 154 mM Na⁺, 144 mM Cl⁻, 10 mM CO³⁻.

**Tab 2:**

| Lösungen für die chromatographische Trennung von Bet v 1 Varianten: | | | | |
|---|---|---|---|---|
| **Chromatog./Protein** | **Probenauftrag** | **Lösung A** | **Lösung B** | **Gradient** |
| **AIEX** | | | | |
| **Fragment 1** | 8 M Urea, 20 mM | 20 mM NaOH, 10 | 20 mM NaOH, | 10 CV |
| | Tris/HCl pH 8.0 | mM NaCl, 11 mM | 500 mM NaCl, 11 | |
| | | NaHCO³ | mM NaHCO³ | |
| **Fragment 2** | s.o. | s.o. | s.o. | s.o. |
| **Trimer** | s.o. | s.o. | 20 mM NaOH, | s.o. |
| | | | 800 mM NaCl, 11 mM NaHCO³ | |

| **HIC** | | | | |
|---|---|---|---|---|
| **Fragment 1** | AIEX Pool auf 2 M | 20 mM NaOH, 2 M | 20 mM NaOH | 10 CV |
| | NaCl einstellen | NaCl, 11 mM | | |
| | | NaHCO³ | | |
| **Fragment 2** | AIEX Pool auf 3 M | 20 mM NaOH, 3 M | 20 mM NaOH | 10 CV |
| | NaCl einstellen | NaCl, 11 nM | | |
| | | NaHCO³ | | |
| **Trimer** | - | - | - | - |

| **Gelfiltration** | | | | |
|---|---|---|---|---|
| **Fragment 1** | HIC-Pool | 10 mM NaOH, 148.4 mM NaCl, 11 mM | - | - |
| | | NaHCO³ | | |
| **Fragment 2** | s.o. | s.o. | - | - |
| **Trimer** | Q-Pool | s.o. | - | - |

Die Lagerung sämtlicher Lösungen und Fraktionen erfolgt bei RT. (CV = Column Volume).

## Patentansprüche

1. Verfahren zur Herstellung der folgenden gereinigten, rekombinanten Varianten des Birkenpollenhauptallergens Bet v 1 in löslicher, biologisch aktiver und in physiologischem, unmittelbar für den medizinischen Einsatz geeigneten Medium, vorliegender Form:
- Trimer,
- Fragment A (Aminosäuren 1-74) und
- Fragment B (Aminosäuren 75-164),
aus nach bakterieller Expression erhaltenen, im Expressionsmedium unlöslichen Proteinaggregaten ("inclusion bodies"),
**dadurch gekennzeichnet, daß** man folgende Schritte ausführt:
(i) Aufnahme der abgetrennten unlöslichen Proteinaggregate in organischen Denaturierungs-Reagenzien,
(ii) Aufreinigung der Lösung aus (i) mittels mindestens eines chromatographischen Schrittes unter Verwendung von im wesentlichen anorganischen ungepufferten alkalischen, salzhaltigen Laufmitteln, wobei es sich bei dem mindestens einen chromatographischen Schritt um eine lonenaustauschromatographie handelt, und
(iii) Neutralisation der nach dem letzten Aufreinigungsschritt erhaltenen, das gelöste, renaturierte und biologisch aktive Protein enthaltenden alkalischen Lösung.

2. Verfahren nach Anspruch 1, **dadurch gekennzeichnet, daß** die besagten rekombinanten Varianten keine oder eine verminderte IgE-Aktivität, jedoch eine erhaltene T-Zell-Aktivität aufweisen.

3. Verfahren nach Anspruch 1 oder 2, **dadurch gekennzeichnet, daß** man als Denaturierungs-Reagenz Harnstoff oder Guanidiniumhydrochlorid einsetzt.

4. Verfahren nach Anspruch 1 bis 3, **dadurch gekennzeichnet, daß** das Denaturierungs-Reagenz aus der Denaturierungslösung entfernt wird, in dem man das gelöste rekombinante Protein an ein Chromatographie-Material bindet und die Denaturierungs-Lösung gegen ein im wesentlichen anorganisches ungepuffertes, alkalisches Laufmittel austauscht, welches eine Salzkonzentration zwischen 5 und 50 mM aufweist, die eine Bindung des Proteins an das besagte Austauschermaterial gewährleistet.

5. Verfahren nach Anspruch 4, **dadurch gekennzeichnet, daß** das gebundene rekombinante Protein durch Erhöhung der Salzkonzentration eluiert und dabei von Verunreinigungen befreit wird.

6. Verfahren nach Anspruch 5, **dadurch gekennzeichnet, daß** die Erhöhung der Salzkonzentration mittels eines Gradienten erfolgt.

7. Verfahren nach einem der Ansprüche 4 bis 6, **dadurch gekennzeichnet, daß** als Chromatographie-Material ein Anionenaustauscher eingesetzt wird.

8. Verfahren nach einem der Ansprüche 4 bis 7, **dadurch gekennzeichnet, daß** man mindestens einen weiteren chromatographischen Reinigungsschritt durchführt.

9. Verfahren nach Anspruch 8, **dadurch gekennzeichnet, daß** als weiterer chromatographischer Schritt eine hydrophobe Interaktionschromatographie und / oder Gelfiltration durchgeführt wird.

10. Verfahren nach einem der Ansprüche 1 bis 9, **dadurch gekennzeichnet, daß** als chromatographisches Laufmittel NaOH, NaHCO₃ und NaCl eingesetzt wird.

11. Verfahren nach einem der Ansprüche 1 bis 10, **dadurch gekennzeichnet, daß** die Neutralisation nach erfolgter Aufreinigung in einem pH-Wert-Bereich zwischen 6,5 bis 8,0 durchgeführt wird.

12. Verfahren nach Anspruch 11, **dadurch gekennzeichnet, daß** zur Neutralisation HCl eingesetzt wird.

## Claims

1. Method for the preparation of the following purified, recombinant variants of the major birch pollen allergen Bet v1 in soluble, biologically active form in a physiological medium which is directly suitable for medical use:
- trimer,
- fragment A (amino acids 1-74) and
- fragment B (amino acids 75-164),
from protein aggregates ("inclusion bodies") obtained after bacterial expression which are insoluble in the expression medium,
**characterised in that** the following steps are carried out:
(i) uptake of the separated-off insoluble protein aggregates in organic denaturing reagents,
(ii) purification of the solution from (i) by means of at least one chromatography step using essentially inorganic unbuffered alkaline, salt-containing eluents, where the at least one chromatography step is ion exchange chromatography, and
(iii) neutralisation of the alkaline solution obtained after the final purification step which comprises the dissolved, renatured and biologically active protein.

2. Method according to Claim 1, **characterised in that** the said recombinant variants have no or reduced IgE activity, but maintained T-cell activity.

3. Method according to Claim 1 or 2, **characterised in that** the denaturing reagent employed is urea or guanidinium hydrochloride.

4. Method according to Claims 1 to 3, **characterised in that** the denaturing reagent is removed from the denaturing solution by binding the dissolved recombinant protein to a chromatography material and exchanging the denaturing solution for an essentially inorganic unbuffered, alkaline eluent which has a salt concentration of between 5 and 50 mM which ensures binding of the protein to the said exchanger material.

5. Method according to Claim 4, **characterised in that** the bound recombinant protein is eluted by increasing the salt concentration and thus freed from impurities.

6. Method according to Claim 5, **characterised in that** the increase in the salt concentration is carried out by means of a gradient.

7. Method according to one of Claims 4 to 6, **characterised in that** the chromatography material employed is an anion exchanger.

8. Method according to one of Claims 4 to 7, **characterised in that** at least one further chromatographic purification step is carried out.

9. Method according to Claim 8, **characterised in that** the further chromatography step carried out is hydrophobic interaction chromatography and/or gel filtration.

10. Method according to one of Claims 1 to 9, **characterised in that** the chromatographic eluent employed is NaOH, NaHCO₃ or Nacl.

11. Method according to one of Claims 1 to 10, **characterised in that** the neutralisation is carried out in a pH range between 6.5 and 8.0 after purification is complete.

12. Method according to Claim 11, **characterised in that** HCl is employed for the neutralisation.

## Revendications

1. Procédé pour la préparation des variants recombinants purifiés de l'allergène de pollen de bouleau majeur Bet v 1 sous forme biologiquement active soluble dans un milieu physiologique qui est directement adapté à une utilisation médicale, comme suit :
- trimère,
- fragment A (acides aminés 1-74) et
- fragment B (acides aminés 75-164),
à partir d'agrégats de protéine ("corps d'inclusion") obtenus après expression bactérienne, qui sont insolubles dans le milieu d'expression,
**caractérisé en ce que** les étapes suivantes sont mises en oeuvre :
(i) soumission des agrégats de protéine insolubles séparés dans des réactifs de dénaturation organique,
(ii) purification de la solution à partir de (i) au moyen d'au moins une étape de chromatographie en utilisant des éluents contenant des sels, alcalins, non tamponnés, essentiellement inorganiques, dans lequel l'au moins une étape de chromatographie est une chromatographie par échange d'ions, et
(iii) neutralisation de la solution alcaline obtenue après l'étape de purification finale qui comprend la protéine renaturée et biologiquement active dissoute.

2. Procédé selon la revendication 1, **caractérisé en ce que** lesdits variants recombinants n'ont pas d'activité IgE ou ont une activité IgE réduite, mais ont une activité de lymphocytes T maintenue.

3. Procédé selon la revendication 1 ou 2, **caractérisé en ce que** le réactif de dénaturation employé est hydrochlorure d'urée ou de guanidinium.

4. Procédé selon les revendications 1 à 3, **caractérisé en ce que** le réactif de dénaturation est enlevé de la solution de dénaturation en liant la protéine recombinante dissoute à un matériau de chromatographie et en échangeant la solution de dénaturation avec un éluent alcalins, non tamponnés, essentiellement inorganique qui a une concentration en sels entre 5 et 50 mM qui assure la liaison de la protéine audit matériau d'échangeur.

5. Procédé selon la revendication 4, **caractérisé en ce que** la protéine recombinante liée est éluée en augmentant la concentration en sels et est ainsi exempte d'impuretés.

6. Procédé selon la revendication 5, **caractérisé en ce que** l'augmentation de la concentration en sels est obtenue au moyen d'un gradient.

7. Procédé selon l'une des revendications 4 à 6, **caractérisé en ce que** le matériau de chromatographie employé est un échangeur d'anions.

8. Procédé selon l'une des revendications 4 à 7, **caractérisé en ce qu'**au moins une étape de purification chromatographique supplémentaire est mise en oeuvre.

9. Procédé selon la revendication 8, **caractérisé en ce que** l'étape de chromatographie supplémentaire mise en oeuvre est une chromatographie par interaction hydrophobe et/ou une filtration sur gel.

10. Procédé selon l'une des revendications 1 à 9, **caractérisé en ce que** l'éluent chromatographique employé est NaOH, NaHCO₃ ou NaCl.

11. Procédé selon l'une des revendications 1 à 10, **caractérisé en ce que** la neutralisation est mise en oeuvre dans une plage de pH entre 6,5 et 8,0 après qu'une purification a été effectuée.

12. Procédé selon la revendication 11, **caractérisé en ce que** HCl est employé pour la neutralisation.
